# EUROPEAN PATENT SPECIFICATION

(11) **EP 0 340 848 B1**
(45) Date of publication and mention of the grant of the patent: **25.08.1993**
(21) Application number: 89201077.8
(22) Date of filing: 26.04.1989
(51) Int. Cl.: C07C 209/48

(54) **Process for the hydrogenation of higher nitriles to amines**
Verfahren zur Hydrogenierung von höheren Nitrilen zu Aminen
Procédé d'hydrogénation de nitriles gras en amines

(30) Priority: 06.05.1988 NL 8801192
(43) Date of publication of application: 08.11.1989
(73) Proprietor: UNILEVER N.V., 3013 AL Rotterdam (NL); UNILEVER PLC, London EC4P 4BQ (GB)
(72) Inventor: Oudejans, Johannes Cornelis, NL-6904 AS Zevenaar (NL)
(74) Representative: Hartong, Richard Leroy

(56) References cited:
- DE-A- 1 518 118
- GB-A- 490 922
- US-A- 4 353 835

## Description

The application relates to a process for the catalytic hydrogenation of higher nitriles to amines, especially to primary amines.

Such processes are already known from the prior art.

EP-A 168 096 (Unilever) discloses a process employing a catalyst consisting of cobalt and diatomaceous earth (kieselguhr) and reports a conversion of nitrile between 70 and 100% in combination with a selectivity to primary amine of 95 and 85%.
Also Raney-nickel catalysts have been proposed for this reaction.

Furthermore US-A 4 353 835 (Gulf Research & Development) discloses to use catalysts based on nickel on a carrier consisting of silica and magnesium oxide for other hydrogenation reactions such as those of unsaturated fatty acids and esters

Finally GB-A 490 922 (Imperial Chemical Industries) discloses the preparation of C6-C10 diamines by hydrogenation of the corresponding dinitriles using catalysts consisting of nickel on a number of carrier materials and mentions in Example 3 the hydrogenation of adiponitrile to hexamethylenediamine with a catalyst consisting of about 30% (w.w.) of nickel on a carrier material consisting of silica and magnesium oxide, prepared from 70 g nickel, 140 g magnesium oxide and about 10 g silica (from metasilicate). The catalyst was dosed in an amount of 3% of nickel per gram of nitrile and resulted in a yield of about 84.5%.

Surprisingly it has now been found that higher nitriles containing 12 to 22 carbon atoms, preferably in a straight chain, can be hydrogenated in good yield to amines (primary as well as secondary) by a catalyst consisting of nickel metal on carrier material containing silica as well as magnesium oxide, in which the catalyst employed according to the present invention contains at least 60 and up to 90 percent by weight of the element nickel, while the atomic ratio of magnesium to nickel usually is between 0.25 and 0.05. More preferably the molar ratio of silica to nickel is between 0.10 to 0.30.

Excellent results are obtained when the catalyst has an average pore radius between 1.5 and 4.0, more preferably between 2.5 and 3.5 nanometers.

Especially with catalysts of the type mentioned above having a pore volume of between 0.20 and 0.80 ml/g of catalyst and an active nickel surface area of between 90 and 150 m²/g nickel (determined by H₂-chemisorption method) it was possible to achieve a high conversion percentage in combination with a high selectivity. As a rule the catalysts employed according to the present invention have a BET-surface area between 100 and 400 m²/g catalyst.

The process according to the present invention is normally carried out under the following conditions: temperature between 120 and 180°C for the preparation of primary amines and between 150 and 250°C for the preparation of secondary amines, a partial hydrogen pressure of between 1 and 3.5 MPa and when applicable a partial ammonia pressure between 1 and 3 MPa.

Suitable nitriles which can be hydrogenated to amines are nitriles derived from higher natural fatty acids such as lauronitrile, palmitonitrile, stearonitrile and oleyl nitrile, especially mixtures of such nitriles or technical grade nitriles can be processed according to the present invention. Tallownitrile is such a technical grade material mainly consisting of stearonitrile, palmitonitrile and oleylnitrile.

Mostly the process according to the present invention is carried out batchwise, but if desirable it can be carried out continuously or semi-continuously. In the case of batchwise processing the amount of catalyst employed lies as a rule between 0.1 and 1.2% (w.w.) calculated as nickel with reference to the amount of nitrile starting material.

### Example 1

300g of tallownitrile were fed into an autoclave with a capacity of 1 litre at room temperature. To the tallownitrile an amount of catalyst was added corresponding to 0.4% of nickel based on the weight amount of nitrile. The autoclave was closed and 15 g ammonia were added by means of a pressure capsule. Subsequently hydrogen was admitted to a total pressure of 2 MPa. and the autoclave was heated in 60 minutes to reaction temperature of 125°C. The total pressure was then about 3 MPa. The contents of the autoclave was stirred and the pressure was kept constant at 3 MPa by supplying additional hydrogen.
After 210 minutes the reaction was terminated and the reaction product was analyzed after the catalyst had been removed. It was found that the nitrile had been converted for 96% into amine, and that the selectivity for primary amine amounted to 98% and that the remaining product (2%) was secondary amine.
The catalyst employed in this example had the following characteristics:
SiO₂/Ni = 0.20 and Mg/Ni = 0.11 (molar ratios), a pore volume of 0.38 ml/g, a mean pore radius of 2.9 nanometers,
an active nickel surface area of 130 m²/g nickel and a BET total surface area of 274 m²/g catalyst.

### Example 2

An amount of 400 g tallownitrile were fed into an autoclave (1 litre capacity) which was equipped with a stirrer. To the nitrile an amount of catalyst had been added which corresponds to 0.25 % (w.w.) calculated on nitrile. Subsequently 10% by weight of liquid ammonia were added to the mixture at room temperature. Hydrogen was then admitted to a pressure of 1.5 MPa and the autoclave was heated in 60 minutes to a temperature of 135°C after which the reaction commenced at a total pressure of 3.5 MPa. This pressure is maintained by supplying hydrogen. After a reaction time of 210 minutes at 145°C the reaction product was analyzed after removal of the catalyst. It was found that 96% of the nitrile had been converted into primary amine and 4% had been converted into secondary amine. No tertiary amine could be detected.
The catalyst employed in this example had the following characteristics:
SiO₂/Ni = 0.19, Mg/Ni = 0.09 (molar ratios), a pore volume of 0.53 ml/g, a mean pore radius of 3.4 nanometers, an active nickel surface area of 110 m²/g nickel and
a BET total surface area of 320 m²/g catalyst.

### Example 3

In an autoclave with a capacity of 1 litre, which was equipped with a stirrer, an amount of 300 g tallow nitrile were fed to which an amount of catalyst had been added corresponding to 0.24 % (w.w.) of nickel calculated on the amount of nitrile feedstock. Subsequently liquid ammonia was added at room temperature in such an amount that after heating the autoclave the partial ammonia pressure was 1.4 MPa. Thereafter hydrogen was admitted so that the pressure in the autoclave increased to 3.5 MPa. Then the temperature was increased to 145°C and the pressure rose to 4.2 MPa. The pressure was kept constant by supplying hydrogen for the reaction period of 210 minutes after which the reaction was discontinued and the reaction product was analyzed after the catalyst had been filtered off. It was determined that 98% of the nitrile had been converted into primary amine and that 2% had been converted into secondary amine. No tertiary amine could be detected.
The catalyst employed in this example had the following characteristics:
SiO₂/Ni = 0.22, Mg/Ni = 0.11 (molar ratios), a pore volume of 0.28 ml/g, a mean pore radius of 3.2 nanometers, an active nickel surface area of 110 m²/g of nickel and a BET total surface area of 262 m²/g catalyst.

### Example 4

This example illustrates the hydrogenation of nitrile leading to secondary amine.
A mixture of 500 g tallow nitrile and 0.05% (w.w.) of catalyst calculated as nickel with reference to the nitrile feedstock was fed into a 1 litre autoclave which was equipped with a stirrer. After closing the autoclave hydrogen was admitted to a pressure of 1.7 MPa and the temperature was raised to 250°C and the pressure rose to 3.0 MPa. Due to the hydrogenation of nitrile to amine the hydrogen pressure dropped continuously and when the pressure had dropped to 1.5 MPa the gaseous phase was vented and subsequently hydrogen was admitted again to the autoclave to a pressure of 3.0 MPa. This venting procedure was repeated until the hydrogen uptake had virtually disappeared and the reaction had been terminated. When this ("regular vent") method is employed ammonia formed by conversion of primary amine to secondary amine is removed by venting the autoclave and the chemical equilibrium of the reaction is shifted as to promote the formation of secondary amines. After termination of the reaction (in 60 minutes) the catalyst is removed by filtration and the filtrate analyzed. The starting tallow nitrile had been 100% converted into amines, 88% into secondary amine, 8% into primary amine and 4% into tertiary amine.
The catalyst employed in this example had the following characteristics:
SiO₂/Ni = 0.22, Mg/Ni = 0.11 (molar ratios), a pore volume of 0,28 ml/g, a mean pore radius of 2.2 nanometers, an active nickel surface area of 110 m²/g nickel and a BET total surface area of 262 m²/g catalyst

## Claims

1. Process for the catalytic hydrogenation of nitrile to amine employing a nickel catalyst upon a carrier containing magnesium oxide and silica characterized in that a nitrile containing 12 to 22 carbon atoms is hydrogenated with a catalyst that contains from 60 to 90% (w.w.) of nickel calculated on the catalyst composition.

2. Process according to claim 1 characterized in that the molar ratio of silica to nickel in the catalyst is between 0.10 and 0.30.

3. Process according to claim 1 or 2 characterized in that the molar ratio of magnesium to nickel in the catalyst is between 0.25 and 0.05.

4. Process according to any of the claims 1 to 3 characterized in that the mean pore radius of the catalyst is between 1.5 and 4.0, preferably between 2.5 and 3.5 nanometers.

5. Process according to any of the preceding claims characterized in that the pore volume of the catalyst is between 0.20 and 0.80 ml/g.

6. Process according to any of the preceding claims characterized in that the active nickel surface area of the catalyst is between 90 and 150 m²/g of nickel.

7. Process according to any of the preceding claims characterized in that the BET total surface of the catalyst is between 100 and 400 m²/g catalyst.

8. Process according to any of the preceding claims characterized in that the hydrogenation is carried out at a temperature of between 120 and 180°C to obtain primary amine or 150 to 250°C to obtain secondary amine at a partial hydrogen pressure of between 1 and 3.5 MPa and when preparing primary amine also a partial ammonia pressure between 1 and 3 MPa.

9. Process according to claim 8 characterized in that secondary amines are prepared in the higher temperature interval characterized in that no ammonia is added to the reaction mixture and that ammonia formed is removed by repeated venting the autoclave during the reaction and pressurizing again with hydrogen.

10. Process according to any of the preceding claims characterized in that a C₁₂-C₂₂ mononitrile is hydrogenated which contains the carbon atoms in a straight chain.

## Patentansprüche

1. Verfahren zur katalytischen Hydrogenierung von Nitril zu Amin unter Verwendung eines Nickelkatalysators auf einem Träger, der Magnesiumoxid und Siliciumdioxid enthält, dadurch gekennzeichnet, daß ein 12 bis 22 Kohlenstoffatome enthaltendes Nitril mit einem Katalysator hydriert wird, der 60 bis 90 % (Gew./Gew.) Nickel, berechnet auf die Katalysatorzusammensetzung, enthält,

2. Verfahren nach Anspruch 1, dadurch gekennzeichnet, daß das molare Verhältnis von Siliciumdioxid zu Nickel im Katalysator zwischen 0,10 und 0,30 liegt.

3. Verfahren nach Anspruch 1 oder 2, dadurch gekennzeichnet, daß das molare Verhältnis von Magnesium zu Nickel im Katalysator zwischen 0,25 und 0,05 liegt.

4. Verfahren nach irgendeinem der Ansprüche 1 bis 3, dadurch gekennzeichnet, daß der mittlere Porenradius des Katalysators zwischen 1,5 und 4,0 nm, vorzugsweise zwischen 2,5 und 3,5 nm, liegt.

5. Verfahren nach irgendeinem der vorhergehenden Ansprüche, dadurch gekennzeichnet, daß das Porenvolumen des Katalysators zwischen 0,20 und 0,80 ml/g liegt.

6. Verfahren nach irgendeinem der vorhergehenden Ansprüche, dadurch gekennzeichnet, daß die aktive Nickeloberfläche des Katalysators zwischen 90 und 150 m²/g Nickel liegt.

7. Verfahren nach irgendeinem der vorhergehenden Ansprüche, dadurch gekennzeichnet, daß die BET-Gesamtoberfläche des Katalysators zwischen 100 und 400 m²/g Katalysator liegt.

8. Verfahren nach irgendeinem der vorhergehenden Ansprüche, dadurch gekennzeichnet, daß die Hydrogenierung zur Gewinnung von primärem Amin bei einer Temperatur zwischen 120 und 180°C oder zur Gewinnung von sekundärem Amin bei 150 bis 250°C bei einem Wasserstoffteildruck zwischen 1 und 3,5 MPa und bei Herstellung von primärem Amin auch bei einem Ammoniakteildruck zwischen 1 und 3 MPa erfolgt.

9. Verfahren nach Anspruch 8, dadurch gekennzeichnet, daß sekundäre Amine im höheren Temperaturintervall hergestellt werden, dadurch gekennzeichnet, daß der Reaktionsmischung kein Ammoniak zugegeben wird und daß der gebildete Ammoniak durch wiederholtes Entspannen des Autoklaven während der Reaktion entfernt und der Druck mit Wasserstoff erneut eingestellt wird.

10. Verfahren nach irgendeinem der vorhergehenden Ansprüche, dadurch gekennzeichnet, daß ein C₁₂-C₂₂-Mononitril hydrogeniert wird, das die Kohlenstoffatome in gerader Kette enthält.

## Revendications

1. Procédé d'hydrogénation catalytique du nitrile en ami ne en utilisant un catalyseur de nickel sur un support contenant de l'oxyde de magnésium et de la silice, caractérisé en ce qu'on hydrogène un nitrile de 12 à 22 atomes de carbone à l'aide d'un catalyseur qui contient de 60 à 90% en poids de nickel par rapport à la composition du catalyseur.

2. Procédé selon la revendication 1, caractérisé en ce que le rapport molaire de la silice au nickel dans le catalyseur est compris entre 0,10 et 0,30.

3. Procédé selon la revendication 1 ou 2, caractérisé en ce que le rapport molaire du magnésium au nickel dans le catalyseur est compris entre 0,25 et 0,05.

4. Procédé selon l'une quelconque des revendications 1 à 3, caractérisé en ce que le rayon moyen des pores du catalyseur est compris entre 1,5 et 4,0, de préférence entre 2,5 et 3,5 nm.

5. Procédé selon l'une quelconque des revendications précédentes, caractérisé en ce que le volume de pores du catalyseur est compris entre 0,20 et 0,80 ml/g.

6. Procédé selon l'une quelconque des revendications précédentes, caractérisé en ce que la surface de contact de nickel actif du catalyseur est comprise entre 90 et 150 m²/g de nickel.

7. Procédé selon l'une quelconque des revendications précédentes caractérisé en ce que la surface totale BET du catalyseur est comprise entre 100 et 400 m²/g de catalyseur.

8. Procédé selon l'une quelconque des revendications précédentes, caractérisé en ce qu'on effectue l'hydrogénation à une température de 120 à 180°C pour obtenir une amine primaire ou de 150 à 250°C pour obtenir l'amine secondaire sous une pression partielle d'hydrogène de 1 à 3,5 MPa et, quand on prépare une amine primaire aussi sous une pression partielle d'ammoniac de 1 à 3 MPa.

9. Procédé selon la revendication 8, caractérisé en ce qu'on prépare les amines secondaires dans la plage des plus hautes températures caractérisé en ce qu'on n'ajoute pas d'ammoniac au mélange de réaction et en ce qu'on élimine l'ammoniac formé par des mises à l'échappement répétées de l'autoclave au cours de la réaction et nouvelle pressurisation avec l'hydrogène.

10. Procédé selon l'une quelconque des revendications précédentes, caractérisé en ce qu'on hydrogène un mononitrile en C₁₂₋₂₂ dont les atomes de carbone sont en chaîne droite.
